# EUROPEAN PATENT APPLICATION

(11) **EP 2 937 399 A1**
(43) Date of publication of application: **28.10.2015**
(21) Application number: 14165385.7
(22) Date of filing: 22.04.2014
(51) Int. Cl.: C09K 11/06, C07C 211/00, H05B 33/10, H01L 31/0224

(54) **Hole-transport materials for organic solar cells or organic optical sensors**

(71) Applicant: BASF SE, 67056 Ludwigshafen (DE)
(72) Inventor: Send, Robert, 76137 Karlsruhe (DE); Bruder, Ingmar, 37271 Neuleiningen (DE); Wonneberger, Henrike, 68165 Mannheim (DE); Michaela, Agari, 69126 Heidelberg (DE); Getautis, Vytautas, LT 51390 Kaunas (LT); Daskeviciene, Maryte, LT 55232 Jonava (LT); Malinauskas, Tadas, LT 49317 Kaunas (LT)

(57) **Abstract**

The present invention relates to hole-transport materials for organic solar cells or organic optical sensors. In particular the present invention relates to a compound of general formula (I) 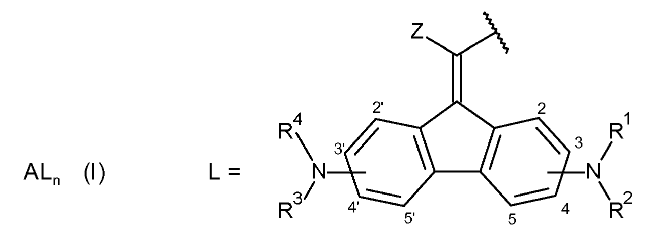 wherein
A is a group which connects all L moieties by a π-conjugated system which is formed by olefinic groups, aromatic groups, heteroaromatic groups, sandwich groups, atoms with unbound electron pairs, or combinations thereof,
Z is hydrogen, an alkyl group, an aryl group, or a heteroaryl group,
R¹, R², R³, R⁴ are independent of each other alkyl groups, aryl groups, or heteroaryl groups,
n is an integer of at least 2.

## Description

The present invention relates to hole-transport materials. The present invention further relates to the use of these materials in organic solar cells or organic optical sensors. The present invention also relates to organic solar cells or organic optical sensors.

Organic solar cells and organic optical sensors are promising alternatives to conventional inorganic devices mostly based on silicon. It is expected that they can be produced at low cost on large-area flexible substrates by means of printing techniques. However, the solar-to-electrical energy conversion efficiencies and the long-term stability have not yet reached those of the conventional inorganic devices. Therefore, intensive research resources are invested with the goal to improve the efficiency and stability of organic solar cells and organic optical sensors.

An organic solar cell containing 2,2',7,7'-tetrakis(N,N-di-p-methoxyphenyl-amine)9,9'-spirobifluorene (OMeTAD) as hole-transport material is disclosed by Bach et al. (Nature Vol. 395, 583-585).

It was an object of the present invention to provide hole-transport materials for organic solar cells or optical sensors with high energy conversion efficiency. These materials should be easily synthesized with cost-effective precursor materials in a short synthesis route and an easy purification procedure. Another object of the present invention is to provide hole-transport materials which have little tendency to crystallize even after long periods of time at elevated temperatures.

The above objects were achieved by a compound of general formula (I) 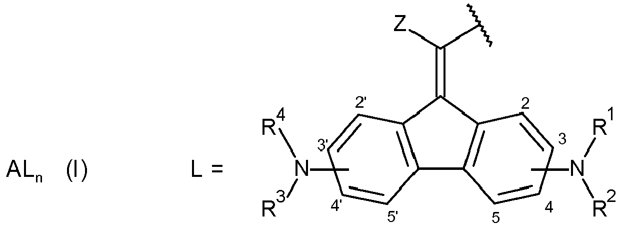 wherein
A is a group which connects all L moieties by a π-conjugated system which is formed by olefinic groups, aromatic groups, heteroaromatic groups, sandwich groups, atoms with unbound electron pairs, or combinations thereof,
Z is hydrogen, an alkyl group, an aryl group, or a heteroaryl group,
R¹, R², R³, R⁴ are independent of each other alkyl groups, aryl groups, or heteroaryl groups,
n is an integer of at least 2.

The present invention further relates to the use of the compound of general formula (I) according to the present invention in organic solar cells or organic optical sensors.

The present invention further relates to an organic solar cell or organic optical sensor comprising a compound of general formula (I) according to the present invention.

Preferred embodiments of the present invention can be found in the description and the claims. Combinations of different embodiments fall within the scope of the present invention.

According to the present invention A in general formula (I) is a group which connects all L moieties by a π-conjugated system which is formed by olefinic groups, aromatic groups, heteroaromatic groups, sandwich compounds, atoms with unbound electron pairs, or combinations thereof. Preferably the π-conjugated system in A is formed by aromatic and/or heteroaromatic groups. More preferably the π-conjugated system in A is formed by benzene.

Olefinic groups can be noncyclic like vinylene and 1,3-butadienyl or cylic like in cyclopentenyl and 1,3-cyclohexadienyl. Noncyclic groups are preferred, in particular vinylene.

Aromatic groups include five-membered rings like the cyclopentadiene anion and six-membered rings like benzene. It is also possible that several aromatic rings are fused such as in naphthalene, anthracene, phenanthrene, the indene anion or the fluorene anion.

Heteroaromatic groups include five-membered rings like furane, pyrrole, thiophene, selenophene, oxazole, isoxazole, imidazole, thiazole, isothiazole, triazole or tetrazole and six-membered rings like pyridine, pyridazine, pyridimine, pyrazine, triazine, or tetrazine. It is also possible that heteroaromatic rings are fused with aromatic rings such as in benzofurane, indole, benzothiophene, benzoselenophene, dibenzofurane, carbazole, dibenzothiophene, dibenzoselenophene, quinoline, isoquinoline or indazine. Another possibility is that two or more heteroaromatic rings are fused such as in furanofurane, pyrrolopyrrole, thiophenothiophene, selenophenoselenophene, furanopyrrole, pyrrolothiophene, dipyrrolopyrrole, dithiophenethiophene.

Sandwich groups are derived from sandwich compounds in which a metal atom is bound to two aromatic or heteroaromatic groups via haptic covalent bonds. Metals can be Cr, Fe, Co, Ni, Pb, Zr, Ru, Rh, Sm, Ti, V, Mo, W, Zn, preferably Fe, Co, Ni, in particular Fe. Aromatic groups in sandwich compounds are preferably cyclopendadiene or its derivatives such as indene or fluorene, cyclopentadiene is preferred. A preferred sandwich group is derived from ferrocene.

Typical atoms with unbound electron pairs are nitrogen, oxygen, phosphor and sulfur, preferably nitrogen.

According to the present invention it is possible that A is formed by a combination of two or more of the same or different groups mentioned before. Examples include several aromatic groups like in biphenyl or terphenyl; several heteroaromatic groups like bipyridine or dithiophene; a combination of olefinic groups with aromatic groups like phenylene-vinylene; a combination of olefinic groups with heteroaromatic groups like in thiophene-vinylene or pyridinevinylene; combination of aromatic and heteroaromatic groups like in phenylthiophene or pyridinylpyrrole; a combination of aromatic groups with atoms with unbound electron pairs like triphenylamine, diphenylnaphthylamine, diphenylether, diphenylthioether or triphenylphosphane; a combination of heteroaromatic groups with atoms with unbound electron pairs like tripyridylamine or dithiophenylthioether; or a combination of several atoms with unbound electron pairs like hydrazine, azo groups, or phosphazides.

According to the present invention the groups L are connected to A in the compound of formula (I) via any atom which is part of the π-conjugated system in A. In the case of benzene this can mean that the groups L in the compound of formula (I) are connected to the 1,2-position, to the 1,3-position or the 1,4 position of benzene if two groups L are present in the compound of formula (I). If more than one aromatic or heteroaromatic group is present in A, the groups L can be connected to the same aromatic or heteroaromatic group or to different ones as long as there is a π-conjugated chain of atoms between the connection sites of L to A.

Various non-conjugated groups can be bound to the π-conjugated system in A as long as A connects all L moieties by a π-conjugated system. These include linear or branched alkyl chains; linear or branched alkoxy chains; polyethers like polyethylene oxide; halogens, namely fluoride, chloride, bromide, iodide; pseudohologens like cyanide, cyanate, thiocyanate; esters; amides; carbonates; carbamates and/or carbamides.

According to the present invention Z is hydrogen, an alkyl group, an aryl group, or a heteroaryl group. Preferably Z is hydrogen. Alkyl groups include linear alkyl groups like methyl, ethyl, n-propyl, n-butyl, n-pentyl, n-hexyl, n-heptyl, n-octyl, n-nonyl, n-decyl, and so on and branched alkyl groups such as iso-propyl, iso-butyl, sec-butyl, tert-butyl, 2-methyl-pentyl, 2-ethyl-hexyl, cyclopropyl, cyclohexyl, indanyl, norbornyl. Preferably, the alkyl groups are C₁-C₂₀ alkyl groups, more preferably C₁-C₁₂ alkyl groups, in particular C₁-C₆ alkyl groups. Alkyl groups in the context of the present invention can also be substituted, for example by halogens like fluoride, chloride, bromide, iodide; by pseudohalogens like cyanide, cyanate, thiocyanate; or by alcohols. It is further possible that one or several methylene units in an alkyl chain are exchanged by oxygen atoms, for example as in a polyethyleneoxide chain. Alkyl groups are preferred.

Aryl groups include phenyl, naphthalyl, anthrancenyl, phenanthrenyl groups. Heteroaromatic groups include pyrryl, furanyl, thienyl, pyridinyl, quinoyl, benzofuryl, benzothiophenyl, thienothienyl. Several of these groups or combinations of these groups are also possible like biphenyl, thienophenyl or furanylthienyl. Aryl groups can be substituted for example by halogens like fluoride, chloride, bromide, iodide; by pseudohalogens like cyanide, cyanate, thiocyanate; by alcohols; alkyl chains or alkoxy chains. Aryl groups are preferred, phenyl is more preferred. The aryl or heteroaryl groups can also be substituted by alkyl groups as described above or by alkoxy groups. Alkoxy groups include methoxy, ethoxy, 1-propoxy, 2-propoxy, 1-butoxy, 2-butoxy or tertbutoxy. Preferably, alkoxy groups are C₁-C₁₂ alkoxy groups, more preferably C₁-C₈ alkoxy groups, even more preferably C₁-C₄ alkoxy groups, in particular methoxy. Alkoxy groups are preferred in particular when attached to an aryl group such as phenyl.

According to the present invention R¹, R², R³, R⁴ are independent of each other alkyl groups, aryl groups, or heteroaryl groups. The same definitions and examples for the alkyl groups, aryl groups, or heteroaryl groups as for Z apply for R¹, R², R³, R⁴. Preferably, R¹, R², R³, R⁴ are independent of each other alkoxyphenyl groups, more preferably all R¹, R², R³, R⁴ are 4-methoxyphenyl groups.

The amino group NR¹R² can be attached to any of the positions 2, 3, 4, 5 and the amino group NR³R⁴ can be attached to any of the positions 2', 3', 4', 5' independent of the position where NR¹R² is attached to. Preferably, NR¹R² is attached to the 3 position. Also preferably, NR³R⁴ is attached to the 3' position. More preferably, NR¹R² is attached to the 3 position and NR³R⁴ is attached to the 3' position.

According to the present invention n is an integer of at least 2. Preferably, n equals 2, 3 or 4, more preferably n equals 2 or 3, in particular n equals 2. The groups L in the compound of general formula (I) can be all the same or different to each other. If n is 3 or larger, it is also possible that two groups L are the same and one or more are different to these. Preferably, all groups L are the same.

The compounds of general formula (I) can be synthesized by base-catalyzed condensation of dihalogen-substitued fluorene and an aldehyde or keto-functionalized group A followed by a transition catalyst catalyzed amination such as a Buchwald-Hartwig coupling according to the following equation:

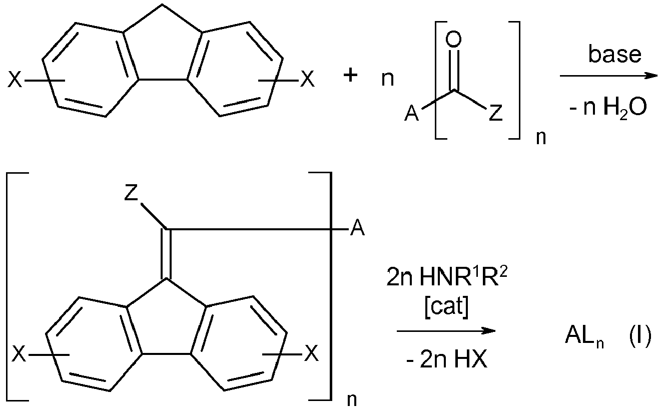

X stands for a halogen, i.e. F, Cl, Br, I, or a halogen equivalent such as triflate, tosylate or mesylate. Preferably, X is Br. The base can be any base which is strong enough to at least partially deprotonate fluorene, preferably NaOH. For the condensation reaction of fluorene with the carbonyl-substituted A any solvent can be used in which the reagents are at least partially soluble. Partially soluble in the present context means a solubility of at least 10 g/I, preferably at least 50 g/I. It is also useful to use a two-phasic mixture, i.e. a hydrophobic solvent like toluene and a hydrophilic solvent like water. In this case it is preferable to use a phase-transfer catalyst, such as tetrabutylammonium bromide. The reaction typically takes place at room temperature within a short time such as 10 minutes to 1 hour. In some cases it is necessary to heat the reaction slightly, e.g. to a temperature of 25 to 80 °C. In case the compound of general formula (I) contains different groups L it is possible to run a sequence of the reaction above each with different fluorene precursors. It may be necessary to use protective groups on the aldehyde or ketone substituted A group. The skilled person knows how to select an appropriate protection group and run the reaction sequence.

The Buchwald-Hartwig coupling is for example described in detail by John F. Hartwig (Angew. Chem. Int. Ed. 37 (1998) 2047-2067). The use of palladium catalyst is preferred, in particular a palladium catalyst containing tri-tert-butylphosphine ligands.

The compound of general formula (I) is particularly suitable as hole-transport materials in organic solar cells or organic optical sensors. Therefore, the present invention relates to the use of the compound of general formula (I) in organic solar cells or organic optical sensors.

The present invention further relates to organic solar cells or organic optical sensors comprising the compound of general formula (I) according to the present invention. Organic solar cells and organic optical sensors are based on the same principle, namely the conversion of incident light to electrical energy. In the case of solar cells the electrical energy is used as power source whereas in the case of optical sensors the electrical energy is used to measure the intensity of the incident light. The following section describes solar cells but equally applies to organic optical sensors which are basically built the same way.

An organic solar cell according to the present invention typically comprises
- a substrate,
- a transparent electrode,
- an electron-transport material,
- the compound of general formula (I) as hole-transport material, and
- a counter electrode.

The components of the solar cell are generally arranged in a stack of layers. Layer in the context of the present invention refers to a thin structure with an arbitrary surface. It may be flat, but in most cases it is very rough. A layer can even form an interpenetrating network with an adjacent layer to increase its contact area to the adjacent layers. The order of the layers can be freely chosen with the provision that the two electrodes are not in direct contact with each other and that the substrate is not in between the two electrodes. An example for the order is
1. the substrate,
2. the transparent electrode,
3. the electron-transport material,
4. the compound of general formula (I) as hole-transport material, and
5. the counter electrode.

According to the present invention the substrate can be made of glass such as low-cost soda glass of high strength or non-alkali glass from which no alkaline elution occurs. Alternatively, a transparent polymer film may be used such as tetraacetyl cellulose (TAC), polyethylene terephthalate (PET), polyethylene naphthalate (PEN), syndiotactic polystyrene (SPS), polyphenylenesulfide (PPS), polycarbonate (PC), polyarylate (PAr), polysulfone (PSF), polyestersulfone (PES), polyimide (PI), polyetherimide (PEI), polycycloolefin such as polynorbornene, or brominated phenoxy resin. Polymer films are preferred, in particular PET, PEN and polynorbornene.

According to the present invention the transparent electrode can be made of a transparent conductive oxide (TCO), such as for example indium-tin composite oxides, tin oxides doped with fluorine, antimony or indium and zinc oxide doped with aluminum. Tin oxide doped with fluorine or indium is preferred. The transparent electrode is generally used in form of a thin film, so that it is sufficiently transparent. Transparent in the present context means that the transmittance of light with a wavelength of 550 nm is at least 50 %, preferably at least 70 %, in particular at least 80 %. Preferably the transparent electrode has a thickness of 0.02 to 10 µm and more preferably from 0.1 to 1 µm.

According to the present invention the electron-transport material can be organic or inorganic. Typical organic electron-transport materials are fullerenes C₆₀, C₇₀, C₇₆, C₈₀, C₈₂, C₈₄, C₈₆, C₉₀ and C₉₄, preferably C₆₀. It is also possible to use substituted fullerenes, in particular [6,6]-phenyl-C₆₁-butyric acid methyl ester (PCBM).

Suitable inorganic electron-transport materials are semi-conductive metal oxides including oxides of titanium, tin, zinc, iron, tungsten, zirconium, hafnium, strontium, indium, cerium, yttrium, lanthanum, vanadium, caesium, niobium or tantalum. Further, composite semiconductors such as M'ₓM²_{y}O_{z} may be used, wherein M¹ and M² are independent of each other a metal atom, O is an oxygen atom, and x, y and z are numbers including 0 which are chosen such that an non-charged molecule is formed. Examples are TiO₂, SnO₂, Fe₂O₃, WO₃, ZnO, Nb₂O₅, SrTiO₃, Ta₂O₅, Cs₂O, zinc stannate, complex oxides such as barium titanate, and binary and ternary iron oxides.

According to the present invention inorganic electron-transport materials are preferred, more preferred are semiconducting metal oxides. Even more preferred are TiO₂, SnO₂, Fe₂O₃, WO₃, ZnO, Nb₂O₅, and SrTiO₃, in particular TiO₂.

The inorganic electron-transport materials are preferably present in amorphous or nanocrystalline form. More preferably, they are present as nanocrystalline porous layers. Such layers have a large surface area leading to effective charge separation of the exciton formed upon light absorption. The inorganic electron-transport materials may also be present in a structured form, such as nanorods.

Preferred methods for producing the semi-conductive metal oxides are sol-gel methods described for example in Materia, Vol. 35, No. 9, Page 1012 to 1018 (1996). The method developed by Degussa Company, which comprises preparing oxides by subjecting chlorides to a high temperature hydrolysis in an oxyhydrogen salt, is also preferred.

In the case of using titanium oxide as the semi-conductive metal oxides, the above-mentioned sol-gel methods, gel-sol methods, high temperature hydrolysis methods are preferably used. Other preferred sol-gel methods are those described in Barbé et al., Journal of American Ceramic Society, Vol. 80, No. 12, Page 3157 to 3171 (1997) and Burnside et al, Chemistry of Materials, Vol. 10, No. 9, Page 2419 to 2425 (1998).

Preferably, the electron-transport material is sensitized with a light-absorbing material to increase light absorption efficiency such as in a dye-sensitized solar cell (DSC). Generally 0.01 to 1 mmol of light-absorbing material is used per 1 g of electron-transport material. Furthermore, the electron or hole transport material may itself absorb light such as in a bulk heterojunction (BHJ) solar cell.

The light-absorbing material in the solar cell according to the present invention can be chosen from a wide variety of substances. Examples are metal complex dyes including Ru(II)-dyes (as for example those in WO 98 / 50 393 - page 8 to 11); indoline dyes (as for example in Adv. Mater. 2005, 17, 813); oxazine dyes (as for example those in US 6 359 211); thiazine dyes (as for example those in US 6 359 211); acridine dyes (as for example those in US 6 359 211); porphyrin dyes; methine dyes such as cyanine dyes, merocyanine dyes (as for example those in WO 2009 / 007 340 page 4 to 7), squarylium dyes; rylene dyes including perylene dyes and naphtalenemonoimid dyes (as for example in those WO 2007 / 054 470 page 13 to 18); or a quinolinium dye (for example those in WO 2009 / 109 499 page 42 to 51). Ru(II) dyes, perylene dyes, naphtalenemonoimid dyes, or quinolinium dyes are preferred.

Other light-absorbing materials include quantum dots like CdSe quantum dots and perovskite absorbers. Perovskite absorbers are preferred. Perovskite absorbers are typically compounds of the general formula (II): EMX₃. E stands for an alkali metal such as Li, Na, K, Rb, Cs; or an ammonium ion in which one or more hydrogen atoms may be exchanged by alkyl chains or acyl groups. Ammonium ions in which one or more hydrogen atoms are exchanged by alkyl chains include monoalkylammonium ions, dialkylammonium ions, trimethylammonium ions, tetramethylammonium ions. Preferably, the alkyl group or groups are independent of each other C₁ to C₆ alkyl groups, in particular methyl or ethyl. Ammonium ions in which one or more hydrogen atoms are exchanged by alkyl chains include amidinium ions, N-alkylamidinium, and imidinium ions, preferably amidinium ions. Preferably, the amidinium or imidinium ion is derived from a C₁ to C₆ carboxamide, in particular from formamide or acetamide. Preferably E is Cs or an ion comprising a positively charged nitrogen atom.

In general formula (II) M stands for a divalent metal atom, preferably for Pb or Sn. X stands for halogens, in particular Cl, Br, I. X in compounds of general formula (II) can contain all the same or different halogens. Specific examples for perovskite absorbers include methyl ammonium lead halogenides, such methyl ammonium lead iodide (CH₃NH₃Pbl₃); methyl ammonium lead mixed halogenides such as CH₃NH₃PbClI₂; formadinium lead halogenides like formadinium lead iodide (HC(NH₂)PbI₃), formadinium lead bromide (HC(NH₂)PbBr₃) or formadinium lead chloride iodide (HC(NH₂)PbCl₂I); or cesium tin iodide (CsSnI₃). Solar cells comprising sensitizers of general formula (II) are sometimes referred to as perovskite-sensitized solar cells (PSC).

The hole transport layer, the sensitizing dye and the electron transport layer can independent of each other be formed by wet chemical processes and by vapor processes. For wet chemical processes the material out of which the layer is formed is dissolved in a solvent, preferably an organic solvent. Any solvent which dissolves the dye is suitable, for example ethanol, acetone, iso-propanol, tetrahydrofurane, dimethylforamide, dimethylacetamide, acetonitrile, methoxyacetonitrile, toluene, N-methylpyrrolidone. The preferred concentration of the sensitizing dye in the solvent is 0.1 to 100 g/I, more preferably it is 1 to 10 g/I. The sensitizing dye dissolved in the solvent is applied to the electron-transport layer by any layer-formation process. These include spin-coating, spray-coating, dip-coating, drop-casting, doctor-blading, slot-die coating, 2D ink jet printing, gravure printing, offset printing, flexo printing, screen printing, or microcontact (wave) printing.

Vapor processes include sublimation, physical vapor deposition, chemical vapor deposition, atomic layer deposition, or direct liquid injection. The material out of which the layer is formed is brought into the gaseous state and deposited onto the other layers. Preferably, the vapor processes are done under reduced pressure such as from 100 to 10⁻⁸ mbar, more preferably from 10 to 10⁻⁵ mbar, in particular 1 to 10⁻² mbar.

After deposition the respective layer is preferably heated. Suitable temperatures are 100 to 600 °C, preferably 200 to 500 °C. Suitable time periods are 10 minutes to 2 hours, preferably 20 minutes to 1 hour.

In the solar cell according to the present invention the compound of general formula (I) acts as hole-transport material. Its layer usually forms an electrical contact with the optionally sensitized electron-transport layer. The compound of general formula (I) can be the only substance in the hole-transport layer or it can be mixed with other substances. An example are dopants which increase the hole conductivity such as N(PhBr)₃SbCl₆, silver-bis-(trifluoromethylsulfonyl)imide or V₂O₅. The layer comprising the hole-transport material containing a dopant is formed by layer-formation process described above wherein the dopant is mixed in the solution for the wet-chemical process or brought into the gas phase together with the hole-transport material.

Furthermore, a solar cell according to the present invention may further comprise a blocking layer between the absorber and the electron-transport layer. Materials suitable for blocking layers include metal oxides, for example TiO₂, SiO₂, Al₂O₃, ZrO₂, MgO, SnO₂, ZnO, Eu₂O₃, Nb₂O₅ or combinations thereof, TiCl₄; or polymers, for example poly(phenylene oxide-co-2-allylphenylene oxide) or poly(methylsiloxane). Details of the preparation of such layers are described in, for example, Electrochimica Acta 40, 643, 1995; J. Am. Chem. Soc 125, 475, 2003; Chem. Lett. 35, 252, 2006; J. Phys. Chem. B, 110, 1991, 2006. Preferably, TiCl₄ is used. The blocking layer is usually dense and compact, and is usually thinner than the electron-transport layer.

One or both electrodes can independent of each other be coated with very thin layers to adjust the work function of the electrodes. These work function adjustment layers may be generated by self-assembled monolayers of organic molecules, preferably aromatic molecules. Examples are thiol-functionalized thiophenes, pentacene derivatives or cyanobenzene derivatives. The self-assembled monolayers are made by immersing the electrode in a solution containing the organic molecule which assembles on the surface. The electrode is removed from the solution and dried.

The solar cell according to the present invention further comprises a counter electrode formed by an electrically conductive material. Examples of the electrically conductive material used for the counter electrically conductive layer include metals such as platinum, gold, silver, copper, aluminum, magnesium, indium or mixtures or alloys thereof, preferably of aluminum and silver; carbon; electrically conductive metal oxides such as indium-tin composite oxides and fluorine-doped tin oxides; mixed inorganic/organic electrodes; polylayer electrodes such as LiF/Al electrodes. Preferred electrically conductive materials are platinum, gold, silver, copper, aluminum or magnesium, more preferred silver or gold.

The thickness of the counter electrode is not particularly limited, preferably it is 3 nm to 10 µm. The counter electrode can be made by applying metal-plating or vapor-depositing such as physical vapor deposition or chemical vapor to deposit the electrically conductive material directly onto the layer it shall contact. It can also be deposited via a printing or coating process from a metal containing ink or paste or a conductive carbon-based formulation.

The solar cells according to the present invention show high light to electrical power conversion efficiency, are easily produced with high reproducibility and show high stability.

### Examples

### Synthesis

### Example 1: Bis{(1,1'-[(2,7-dibromo-9H-fluoren-9-ylidene)methyl]cyclopentadienyl}iron (1)

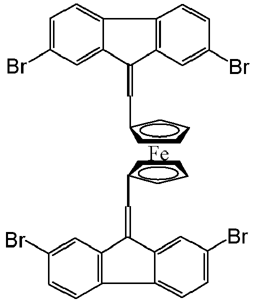

To 1,1'-ferrocene dicarboxaldehyde (0.92 g, 3.8 mmol), 2,7-dibromofluorene (3.09 g, 9.5 mmol) and tetrabutylammonium bromide (0.44 g, 1.4 mmol) dissolved in toluene (20 mL), aqueous 40% NaOH solution (20 mL) was added and the obtained mixture was stirred at room temperature for 15 min. Red brown crystals of 1 (3 g, 92%), formed upon standing, were filtered off and washed with water until neutral and with 2-propanol. The product was recrystallized from toluene:dioxane = 2:1, m.p. >400 °C.
¹H-NMR (400 MHz, DMSO-d₆, few drops of CS₂): δ = 8.43 - 8.20 (m, 2H), 8.05 - 7.08 (m, 12 H), 4.94 (s, 4H), 4.74 (s, 4H).
Anal. calcd for C₃₈H₂₂Br₄Fe: C, 53.44; H, 2.60; found: C, 53.66; H, 2.89.

### Example 2: Bis[1,1'-({2,7-bis[bis(4-methoxyphenyl)amino]-9H-fluoren-9ylidene}methyl)cyclopentadienyl]iron (HTM-1)

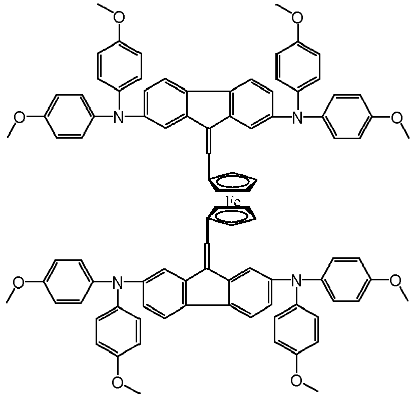

A solution of 1 (1.07 g, 1.25 mmol), 4,4'dimethoxydiphenylamine (1.72 g, 7.5 mmol) in anhydrous toluene (14 mL) was purged with argon for 20 minutes. Afterwards, palladium(II) acetate (5.6 mg, 0.025 mmol), tri-tert-butylphosphonium tetrafluoroborate (10 mg, 0.03 mmol) and sodium tert-butoxide (0.72 g, 7.5 mmol) were added and the solution was refluxed under argon atmosphere for 7.5 hours. After cooling to room temperature, reaction mixture was filtered through Celite, 50 mL of distilled water were added and extraction was done with ethyl acetate. The combined organic layer was washed with distilled water, dried over anhydrous Na₂SO₄, filtered and solvent evaporated. The crude product was purified by column chromatography using 1:1 v/v THF/n-hexane as an eluent. The obtained product was precipitated from 20% solution in THF into 10-fold excess of n-hexane. The precipitate was filtered off and washed with n-hexane to collect HTM-1 as a red solid (1.23 g, 68 %).
¹H-NMR (400 MHz, CDCl₃): δ = 8.10-7.75 (m, 2H), 7.65 (s, 2H), 7.38 (d, J = 7.9 Hz, 4H), 7.306.62 (m, 38H), 4.21 (s, 4H), 4.04 (s, 4H), 3.86 - 3.64 (m, 24H).
¹³C-NMR (100 MHz, CDCl₃): δ = 156.19, 155.55, 155.26, 148.96, 147.34, 147.09, 141.75, 141.55, 141.19, 140.51, 137.76, 136.74, 135.69, 134.05, 133.81, 132.68, 126.72, 126.20, 125.64, 125.42, 122.74, 121.19, 120.05, 119.36, 119.29, 118.62, 116.75, 114.96, 114.72, 114.65, 114.32, 81.71, 71.82, 71.20, 55.61, 55.57, 55.53.
Anal. calcd for C₉₄H₇₈FeN₄O₈: C, 78.00; H, 5.43; N, 3.87; found: C, 78.11; H, 5.36; N, 3.74.

### Example 3: Bis[1,1'-({2,7-bis[(4-methoxy-3-methylphenyl)(4-methoxyphenyl)amino]-9H-fluoren-9-ylidene}methyl)cyclopentadienyl]iron (HTM-2)

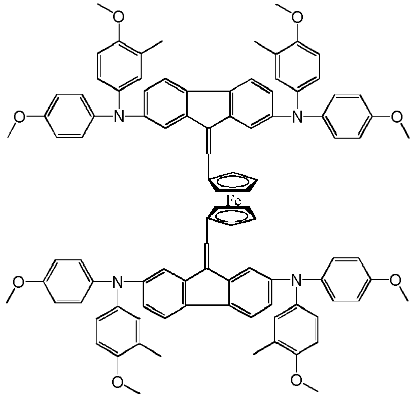

A solution of 1 (1.07 g, 1.25 mmol), 4,4'-dimethoxy-3-methyldiphenylamine (1.825 g, 7.5 mmol) in anhydrous toluene (14 mL) was purged with argon for 20 minutes. Afterwards, palladium(II) acetate (5.6 mg, 0.025 mmol), tri-tert-butylphosphonium tetrafluoroborate (10 mg, 0.03 mmol) and sodium tert-butoxide (0.72 g, 7.5 mmol) were added and the solution was refluxed under argon atmosphere for 7.5 hours. After cooling to room temperature, reaction mixture was filtered through Celite, 50 mL of distilled water were added and extraction was done with ethyl acetate. The combined organic layer was washed with distilled water, dried over anhydrous Na₂SO₄, filtered and solvent evaporated. The crude product was purified by column chromatography using 2:23 v/v acetone/n-hexane as an eluent. The obtained product was precipitated from 20% solution in THF into 10-fold excess of methanol. The precipitate was filtered off and washed with methanol to collect HTM-2 as a red solid (1.07 g, 57 %).
¹H-NMR (400 MHz, CDCl₃): δ = 7.63 (d, J = 2.0 Hz, 2H), 7.38 (d, J = 8.3 Hz, 4H), 7.24 (d, J = 2.0 Hz, 2H), 7.03 (d, J = 9.0 Hz, 4H), 6.99 (d, J = 9.0 Hz, 4H), 6.96 - 6.76 (m, 18H), 6.74 (d, J = 9.0 Hz, 4H), 6.70 (d, J = 8.6 Hz, 2H), 6.62 (d, J = 8.7 Hz, 2H), 4.21 (s, 4H), 4.02 (s, 4H), 3.74 (m, 24H), 2.12 (d, J = 12.3 Hz, 12H).
¹³C-NMR (100 MHz, CDCl₃): δ = 155.43, 155.12, 153.92, 153.65, 147.49, 147.20, 141.98, 141.65, 141.30, 141.17, 141.09, 137.73, 133.96, 133.89, 132.64, 127.76, 127.60, 127.52, 127.25, 126.09, 125.51, 125.33, 123.40, 122.84, 121.23, 119.33, 119.25, 118.64, 114.68, 114.61, 114.42, 110.75, 110.73, 81.77, 71.69, 71.23, 55.64, 55.60, 55.58, 55.54, 16.52, 16.42.
Anal. calcd for C₉₄H₈₆FeN₄O₈: C, 78.28; H, 5.77; N, 3.73; found: C, 78.45; H, 5.89; N, 3.74.

### Example 4: 9,9'-([2,5-bis(hexyloxy)benzene-1,4-diyl]bis{(E)ethene-2,1-diyl[2,5-bis(hexyloxy)benzene-4,1-diyl]methylylidene})bis(2,7-dibromo-9H-fluorene) (2)

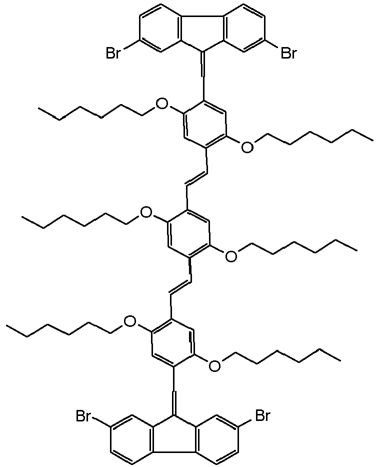

To 2,5-bis(hexyloxy)-1,4-bis[(2,5-bis(hexyloxy)-4-formylphenylenevinylene]benzene (1.2 g, 1.3 mmol), 2,7-dibromofluorene (1.04 g, 3.2 mmol) and tetrabutylammonium bromide (0.15 g, 0.05 mmol) dissolved in toluene (12 mL), aqueous 40% NaOH solution (12 mL) was added and the obtained mixture was refluxed for 1 hour. The mixture was extracted with toluene and water until neutral, the organic layer was dried over anhydrous Na₂SO₄, filtered off and the solvent was evaporated. The crude product was purified by column chromatography using 1:1 v/v toluene/n-hexane as an eluent to collect 2 as a red solid (1.6 g, 81 %), m. p.= 215-217°C (tetrahydrofurane:toluene = 2:1).
¹H-NMR (400 MHz, CDCl₃): δ = 8.16 (s, 2H), 7.91 (s, 2H), 7.78 (s, 2H), 7.68 - 7.51 (m, 8H), 7.46 (t, J = 9.6 Hz, 4H), 4.10 (t, J = 5.8 Hz, 8H), 4.00 (t, J = 6.4 Hz, 4H), 1.98-1.76 (m, 12H), 1.65-1.23 (m, 36 H), 1.00-0.81 (m, 18H).
¹³C-NMR (100 MHz, CDCl₃): δ = 152.19, 151.34, 150.47, 141.77, 139.09, 138.35, 136.70, 133.23, 131.21, 130.84, 129.88, 127.60, 126.70, 124.73, 124.27, 123.83, 123.34, 121.30, 121.14, 120.98, 120.67, 115.40, 110.67, 110.02, 69.96, 69.58, 69.41, 31.84, 31.75, 29.65, 29.48, 26.14, 26.07, 26.04, 22.82, 22.78, 14.25, 14.18.
Anal. calcd for C₈₆H₁₀₂Br₄O₆: C; 66.58; H, 6.63; found: C, 66.75; H, 6.51.

### Example 5: 9,9'-([2,5-bis(hexyloxy)benzene-1,4-diyl]bis{(E)ethene-2,1-diyl[2,5-bis(hexyloxy)benzene-4,1-diyl]methylylidene})bis[N,N,N',N'-tetrakis(4-methoxyphenyl)-9H-fluorene-2,7-diamine] (HTM-3)

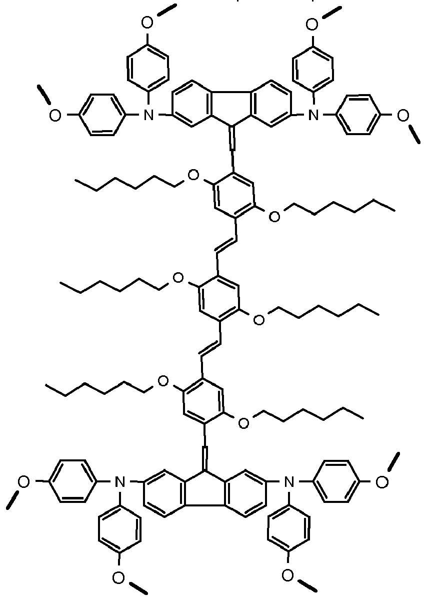

A solution of 2 (0.62 g, 0.4 mmol), 4,4'dimethoxydiphenylamine (0.55 g, 2.4 mmol) in anhydrous toluene (8 mL) was purged with argon for 20 minutes. Afterwards, palladium(II) acetate (4.9 mg, 0.02 mmol), tri-tert-butylphosphonium tetrafluoroborate (13.2 mg, 0.04 mmol) and sodium tert-butoxide (0.23 g, 2.4 mmol) were added and the solution was refluxed under argon atmosphere for 6 hours. After cooling to room temperature, reaction mixture was filtered through Celite, 50 mL of distilled water were added and extraction was done with ethyl acetate. The combined organic layer was washed with distilled water, dried over anhydrous Na₂SO₄, filtered and solvent evaporated. The crude product was purified by column chromatography using 1:1:18 v/v acetone/THF/n-hexane as an eluent. The obtained product was precipitated from 20% solution in acetone into 10-fold excess of methanol. The precipitate was filtered off and washed with methanol to collect HTM-3 as a red brown solid (0.7 g, 82 %).
¹H-NMR (400 MHz, CDCl₃): δ = 7.53-6.62 (m, 56H), 4.09 (t, J= 6.5 Hz, 4H), 3.91 (t, J = 6.5 Hz, 4H), 3.80 (s, 12H), 3.76-3.64 (m, 16H), 1.96-1.82 (m, 4H), 1.82-1.17 (m, 44H), 0.96-0.76 (m, 18H).
¹³C-NMR (100 MHz, CDCl₃): δ = 151.21, 150.41, 141.05, 138.16, 136.12, 128.18, 127.58, 125.79, 123.62, 123.53, 118.16, 114.82, 114.66, 114.51, 110.61, 110.51, 69.62, 69.54, 69.20, 68.63, 55.61, 55.43, 31.84, 31.81, 31.61, 29.83, 29.67, 29.59, 29.32, 27.93, 26.10, 26.09, 25.81, 22.81, 22.70, 14.20, 14.17.
Anal. calcd for C₁₄₂H₁₅₈N₄O₁₄: C, 79.52; H, 7.43; N, 2.61; found: C, 79.30; H, 7.61; N, 2.79.

### Example 6: 4-[(2,7-dibromo-9H-fluoren-9-ylidene)methyl]-N-{4-[(2,7-dibromo-9H-fluoren-9-ylidene)methyl]phenyl}-N-phenylaniline (3)

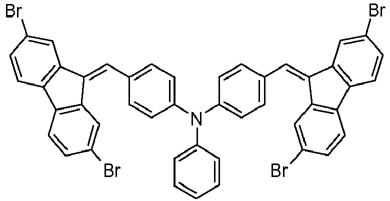

To 4,4'-diformyltriphenylamine (1.50 g, 4.97 mmol), 2,7-dibromofluorene (4.03 g, 12.44 mmol) and tetrabutylammonium bromide (0.58 g, 1.79 mmol) dissolved in toluene (28 mL), aqueous 40% NaOH solution (28 mL) was added and the obtained mixture was refluxed for 30 min. After cooling to room temperature, 50 mL of distilled water were added and extraction was done with ethyl acetate. The combined organic layer was washed with distilled water, dried over anhydrous Na₂SO₄ and filtered. After evaporation of the solvent, the crude product was purified by column chromatography using 3:22 v/v acetone/n-hexane as an eluent to collect 3 as a light orange crystals (1.90 g, 42%), m.p. 176-177 °C.
¹H-NMR (400 MHz, CDCl₃): δ = 7.86 (dd, J=6.9 Hz, J=1.4 Hz, 2H), 7.75 (d, J=1.7 Hz, 2H), 7.67-7.58 (m, 4H), 7.56-7.23 (m, 18H), 7.21-7.09 (m, 1 H);
¹³C-NMR (100 MHz, CDCl₃): δ = 147.88, 146.68, 142.22, 141.17, 138.84, 138.05, 137.43, 136.71, 135.22, 133.69, 131.16, 130.85, 130.63, 129.89, 129.84, 129.68, 127.81, 127.29, 125.58, 123.47, 123.28, 121.81, 121.12, 120.98, 120.90, 120.70.
Anal. calcd for C₄₆H₂₇Br₄N: C, 60.49; H, 2.98, N, 1.53; found: C, 60.69; H, 2.76; N, 1.64.

### Example 7: 9,9'-[(phenylimino)bis(benzene-4,1-diylmethylylidene)]bis[N,N,N',N'-tetrakis(4-methoxyphenyl)-9H-fluorene-2,7-diamine] (HTM-4)

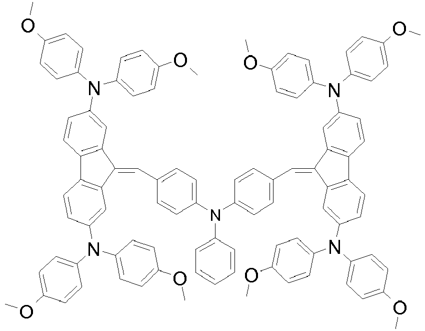

A mixture of 3 (1.00 g, 1.09 mmol), 4,4'dimethoxydiphenylamine (1.5 g, 6.57 mmol) and toluene (18 mL) was purged with argon for 30 minutes. Afterwards, palladium(II) acetate (0.005 g, 0.02 mmol), tri-tert-butylphosphonium tetrafluoroborate (0.009 g, 0.029 mmol) and sodium tert-butoxide (0.63 g, 6.57 mmol) were added and the reaction mixture was refluxed under argon atmosphere for 5 hours. After cooling to room temperature, reaction mixture was filtered through Celite, 50 mL of distilled water were added and extraction was done with ethyl acetate. The organic layer was dried over anhydrous Na₂SO₄, filtered, and solvent evaporated. The crude product was purified by column chromatography using 3:22 v/v acetone/n-hexane as an eluent to collect HTM-4 as a dark orange solid (1.03 g, 63 %).
¹H-NMR (400 MHz, CDCl₃): δ = 7.67-6.44 (m, 59H), 3.78 (s, 12H), 3.58 (s, 12H);
¹³C-NMR (100 MHz, CDCl₃): δ = 147.05, 146.89, 140.92, 130.39, 130.33, 129.29, 124.62, 123.58, 123.25, 119.51, 114.44, 113.98, 55.45, 55.32.
Anal. calcd for C₁₀₂H₈₃N₅O₈: C, 81.31; H, 5.55, N, 4.65; found: C, 81.15; H, 5.76; N, 4.54.

### Example 8: 4-[(2,7-dibromo-9H-fluoren-9-ylidene)methyl]-N-{4-[(2,7-dibromo-9H-fluoren-9-ylidene)methyl]phenyl}-N-(4-methylphenyl)aniline (4)

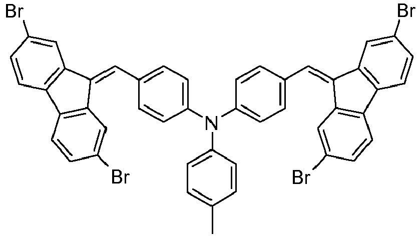

To 4,4'-[(4-methylphenyl)imino]dibenzaldehyde (1.50 g, 4.76 mmol), 2,7-dibromofluorene (3.85 g, 11.89 mmol) and tetrabutylammonium bromide (0.55 g, 1.71 mmol) dissolved in toluene (27 mL), aqueous 40% NaOH solution (27 mL) was added and the obtained mixture was refluxed for 15 min. After cooling to room temperature, 50 mL of distilled water were added and extraction was done with ethyl acetate. The combined organic layer was washed with distilled water, dried over anhydrous Na₂SO₄ and filtered. After evaporation of the solvent, the crude product was purified by column chromatography using 3:22 v/v acetone/n-hexane as an eluent to collect compound 4 as orange crystals (3.68 g, 84%), m.p. 209-210 °C.
¹H-NMR (400 MHz, CDCl₃): δ = 7.87 (dd, J=5.3 Hz, J=1.5 Hz, 4H), 7.62 (s, 2H), 7.57-7.36 (m, 12H), 7.30-7.23 (m, 4H), 7.20 (s, 4H), 2.37 (s, 3H);
¹³C-NMR (100 MHz, CDCl₃): δ = 147.98, 144.00, 141.22, 138.22, 138.07, 136.68, 134.48, 133.50, 131.12, 130.78, 130.61, 130.38, 129.94, 129.52, 127.26, 126.01, 123.44, 123.01, 121.09, 120.97, 120.88, 120.69, 20.96.
Anal. calcd for C₄₇H₂₉Br₄N: C, 60.87; H, 3.15, N, 1.51; found: C, 60.71; H, 3.27; N, 1.62.

### Example 9: 9,9'-{[(4-methylphenyl)imino]bis(benzene-4,1-diylmethylylidene)}bis[N,N,N',N'-tetrakis(4-methoxyphenyl)-9H-fluorene-2,7-diamine] (HTM-5)

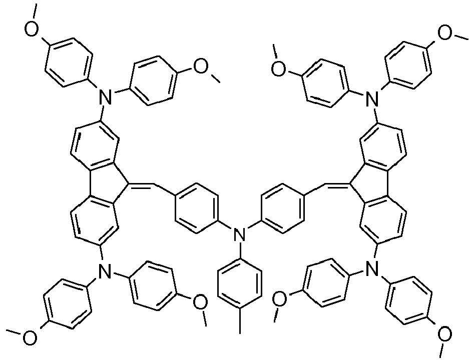

A solution of 4 (1.00 g, 1.08 mmol), 4,4'dimethoxydiphenylamine (1.48 g, 6.47 mmol) and toluene (12 mL) was purged with argon for 30 minutes. Afterwards, palladium(II) acetate (0.005 g, 0.02 mmol), tri-tert-butylphosphonium tetrafluoroborate (0.009 g, 0.029 mmol) and sodium tert-butoxide (0.62 g, 6.47 mmol) were added and the solution was refluxed under argon atmosphere for 5 hours. After cooling to room temperature, reaction mixture was filtered through Celite, 50 mL of distilled water were added and extraction was done with ethyl acetate. The organic layer was dried over anhydrous Na₂SO₄, filtered, and solvent evaporated. The crude product was purified by column chromatography using 3:22 v/v acetone/n-hexane as an eluent to collect HTM-5 as a dark orange solid (1.52 g, 92 %).
¹H-NMR (400 MHz, CDCl₃): δ = 7.66-7.30 (m, 10H), 7.27 (s, 2H), 7.22 (d, J=8.4 Hz, 4H), 7.15-7.68 (m, 22H), 6.82 (d, J=8.4 Hz, 8H), 6.70 (d, J=8.4 Hz, 12H), 3.78 (s, 12H), 3.58 (s, 12H), 2.34 (s, 3H);
¹³C-NMR (100 MHz, CDCl₃): δ = 147.04, 144.38, 140.96, 137.42, 133.31, 130.29, 130.00, 129.91, 127.02, 125.31, 122.98, 119.49, 114.51, 113.98, 55.43, 55.29, 20.90.
Anal. calcd for C₁₀₃H₈₅N₅O₈: C, 81.35; H, 5.63, N, 4.60; found: C, 81.58; H, 5.51; N, 4.42.

### Example 10: 9,9'-(benzene-1,4-diyldimethylylidene)bis(2,7-dibromo-9H-fluorene) (5)

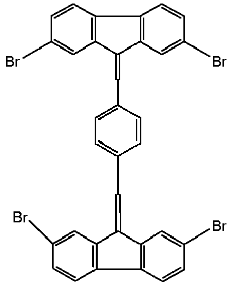

To terephthaldicarboxaldehyde (0.5 g, 3.73 mmol), 2,7-dibromofluorene (3.02, g 9.33 mmol) and tetrabutylammonium bromide (0.43 g, 1.34mmol) dissolved in toluene (18 mL), aqueous 40% NaOH solution (18 mL) was added and the obtained mixture was refluxed for 10 min. Yellow crystals of 5 (2.5 g, 90 %), formed upon standing, were filtered off and washed with hot dimethylformamide and later with diethyl ether. The product was recrystallized from dimethylformamide, m.p. 356-358°C.
¹H-NMR (400 MHz, THF-d₈, few drops of CS₂): δ = 8.01 (d, J = 1.6 Hz, 2H), 7.91 (s, 2H), 7.84 (d, J = 1.7 Hz, 2H), 7.71 (s, 4H), 7.65 - 7.55 (m, 4H), 7.47 - 7.34 (m, 4H).
Anal. calcd for C₃₄H₁₈Br₄: C 54.73; H 2.43; found: C 54.49; H 2.57.

### Example 11: 9,9'-(benzene-1,4-diyldimethylylidene)bis[N,N,N',N'-tetrakis(4-methoxyphenyl)-9H-fluorene-2,7-diamine] (HTM-6)

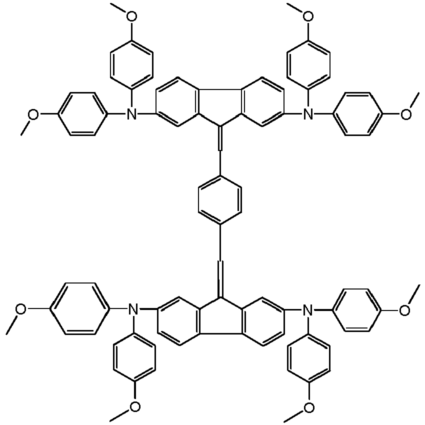

A solution of 5 (0.45 g, 0.6 mmol), 4,4'dimethoxydiphenylamine (0.83 g, 3.6 mmol) in anhydrous toluene (12 mL) was purged with argon for 20 minutes. Afterwards, palladium(II) acetate (7.41 mg, 0.03 mmol), tri-tert-butylphosphonium tetrafluoroborate (19.74 mg, 0.07 mmol) and sodium tert-butoxide (0.35 g, 3.6 mmol) were added and the solution was refluxed under argon atmosphere for 6 hours. After cooling to room temperature, reaction mixture was filtered through Celite, 50 mL of distilled water were added and extraction was done with ethyl acetate. The combined organic layer was washed with distilled water, dried over anhydrous Na₂SO₄, filtered and solvent evaporated. Obtained product was recrystallized from THF, filtered off and washed with tetrahydrofurane and diethyl ether to collect HTM-6 as a red brown solid (0.35 g, 43 %), m. p. = 289-290°C (THF).
¹H-NMR (400 MHz, CDCl₃): δ = 7.44 - 7.32 (m, 6H), 7.24 (d, J = 3.0 Hz, 2H), 7.07 (d, J = 8.9 Hz, 8H), 7.00 - 6.77 (m, 26H), 6.58 (d, J = 8.9 Hz, 8H), 3.78 (s, 12H), 3.54 (s, 12H).
¹³C-NMR (100 MHz, CDCl₃): δ = 155.49, 155.46, 147.32, 147.23, 141.71, 141.07, 140.65, 137.58, 136.49, 135.63, 134.18, 133.44, 128.68, 127.05, 126.21, 125.85, 123.01, 121.29, 119.39, 119.19, 118.56, 114.77, 114.64, 114.45, 55.62, 55.21.
Anal. calcd for C₉₀H₇₄N₄O₈: C, 80.69; H, 5.57; N, 4.18; found: C, 80.65; H, 5.73; N, 4.29.

### Example 12: 9,9'-(benzene-1 ,3-diyldimethylylidene)bis(2,7-dibromo-9H-fluorene) (6)

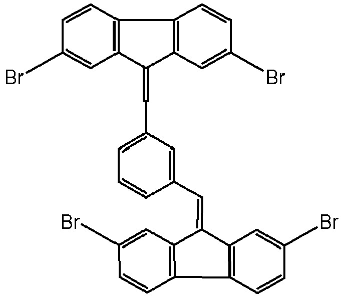

To isophthalaldehyde (0.5 g, 3.73 mmol), 2,7-dibromofluorene (3.02, g 9.33 mmol) and tetrabutylammonium bromide (0.43 g, 1.34mmol) dissolved in toluene (18 mL), aqueous 40% NaOH solution (18 mL) was added and the obtained mixture was refluxed for 2.5 h. Yellow green crystals of 6 (0.8 g, 29 %), formed upon standing, were filtered off and washed with hot dimethylformamide and later with diethyl ether. The product was recrystallized from tetrahydrofurane, m.p. 278-280°C.
¹H-NMR (400 MHz, THF-d₈, few drops of CS₂): δ = 8.00 (d, J = 1.6 Hz, 2H), 7.90 (s, 2H), 7.67 (s, 1 H), 7.65 - 7.54 (m, 7H), 7.47 - 7.32 (m, 4H), 7.13 - 6.93 (m, 2H).
¹³C-NMR (100 MHz, THF-d₈, few drops of CS₂): δ = 138.06, 132.69, 132.34, 130.97, 130.38, 130.18, 129.83, 129.32, 128.37, 125.12, 122.50, 122.29, 121.83.
Anal. calcd for C₃₄H₁₈Br₄: C, 54.73; H, 2.43; found: C, 54.51; H, 2.62.

### Example 13: 9,9'-(benzene-1,3-diyldimethylylidene)bis[N,N,N',N'-tetrakis(4-methoxyphenyl)-9H-fluorene-2,7-diamine] (HTM-7)

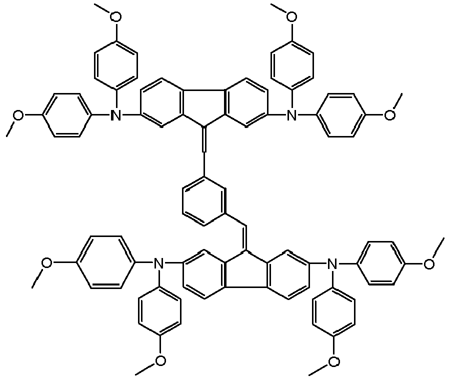

A solution of 6 (0.45 g, 0.6 mmol), 4,4'dimethoxydiphenylamine (0.83 g, 3.6 mmol) in anhydrous toluene (12 mL) was purged with argon for 20 minutes. Afterwards, palladium(II) acetate (7.41 mg, 0.03 mmol), tri-tert-butylphosphonium tetrafluoroborate (19.74 mg, 0.07 mmol) and sodium tert-butoxide (0.35 g, 3.6 mmol) were added and the solution was refluxed under argon atmosphere for 5 h. After cooling to room temperature, reaction mixture was filtered through Celite, 50 mL of distilled water were added and extraction was done with ethyl acetate. The combined organic layer was washed with distilled water, dried over anhydrous Na₂SO₄, filtered and solvent evaporated. The crude product was purified by column chromatography using 1:7 v/v acetone/n-hexane as an eluent. The obtained product was precipitated from 20% solution in tetrahydrofuran into 10-fold excess of toluene. The precipitate was filtered off and washed with diethylether to collect HTM-7 as a red solid (0.54 g, 67 %).
¹H-NMR (400 MHz, CDCl₃): δ = 7.40 - 7.28 (m, 6H), 7.21 (s, 1 H), 7.13 - 7.02 (m, 10H), 7.01 - 6.92 (m, 6H), 6.90 - 6.76 (m, 18H), 6.74 (t, J = 7.7 Hz, 1 H), 6.65 (d, J = 8.9 Hz, 8H), 3.72 (s, 12H), 3.70 (s, 12H).
¹³C-NMR (100 MHz, CDCl₃): δ = 155.66, 155.52, 147.33, 147.16, 141.65, 141.00, 140.60, 137.56, 136.49, 136.33, 134.13, 133.29, 128.86, 128.22, 127.88, 127.01, 126.52, 126.00, 122.64, 120.96, 119.28, 116.65, 114.76, 114.63, 114.20, 55.56, 55.49.
Anal. calcd for C₉₄H₇₄N₄O₈:. Found: C, 80.69; H, 5.57; N, 4.18; found: C, 80.47; H, 5.72; N, 4.01.

### Example 14: 9,9'-(benzene-1,2-diyldimethylylidene)bis(2,7-dibromo-9H-fluorene) (7)

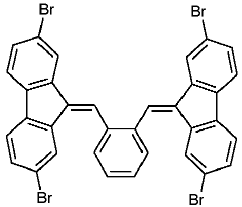

To o-phthalaldehyde (0.5 g, 3.73 mmol), 2,7-dibromofluorene (3.02, g 9.33 mmol) and tetrabutylammonium bromide (0.43 g, 1.34mmol) dissolved in toluene (18 mL), aqueous 40% NaOH solution (18 mL) was added and the obtained mixture was refluxed for 2.5 h. To the reaction mixture ethanol was added and formed crystals were filtered off, washed with water, ethanol and finally with ethylacetate to isolate 7 as slightly yellow crystals (1.5 g, 54 %), m.p. 333-334 °C.
¹H-NMR (700 MHz, DMSO-d₆): δ = 8.26 - 8.01 (m, 4H), 7.70 (s, 2H), 7.25 (t, J = 7.6 Hz, 2H), 7.21 - 6.88 (m, 10H);
IR (KBr)v (cm⁻¹): 3053, 3021 (CHₐᵣ); 1598, 1571, 1452, 1411, 1399 (C=C); 815, 804, 784 (CH=CH of 1,2-disubstituted benzene); 657 (C-Br).
Anal. calcd for C₃₄H₁₈Br₄: C, 54.73; H, 2.43; found: C, 54.95; H, 2.33.

### Example 15: 9,9'-(benzene-1,2-diyldimethylylidene)bis[N,N,N',N'-tetrakis(4-methoxyphenyl)-9H-fluorene-2,7-diamine] (HTM-8)

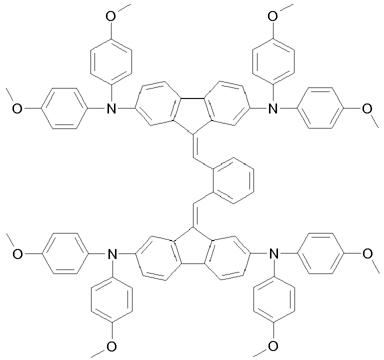

A solution of 7 (0.45 g, 0.60 mmol), 4,4'dimethoxydiphenylamine (0.83 g, 3.6 mmol) and toluene (12 mL) was purged with argon for 30 minutes. Afterwards, palladium(II) acetate (0.003 g, 0.01 mmol), tri-tert-butylphosphonium tetrafluoroborate (0.005 g, 0.02 mmol) and sodium tert-butoxide (0.35 g, 3.60 mmol) were added and the solution was refluxed under argon atmosphere for 5 hours. After cooling to room temperature, reaction mixture was filtered through Celite, 40 mL of distilled water were added and extraction was done with ethyl acetate. The organic layer was dried over anhydrous Na₂SO₄, filtered, and solvent evaporated. The crude product was purified by column chromatography using 0.5:10:14.5 v/v acetone/n-hexane/toluene as an eluent to collect HTM-8 as a light brown red solid (0.34 g, 42 %). ¹H-NMR (300 MHz, CDCl₃): δ = 7.39 (s, 1 H), 7.37 (d, J=2.8 Hz, 2H), 7.34 (s, 1 H), 7.20 (d, J=2.0 Hz, 2H), 7.12 (s, 2H), 7.10-6.98 (m, 10H), 6.97-6.91 (m, 4H), 6.88 (d, J=9.0 Hz, 8H), 6.80 (d, J=9.0 Hz, 8H), 6.76-6.69 (m, 12H), 3.81 (s, 12H), 3.77 (s, 12H);
¹³C-NMR (75 MHz, CDCl₃): δ = 155.32, 147.12, 141.35, 141.22, 140.20, 137.59, 136.25, 134.93, 134.38, 133.05, 129.18, 123.34, 125.97, 125.84, 125.78, 122.37, 121.75, 119.16, 119.02, 118.45, 114.59, 114.47, 114.16, 55.41.
Anal. calcd for C₉₀H₇₄N₄O₈: C, 80.70; H, 5.57, N, 4.18; found: C, 80.52; H, 5.41; N, 4.29.

### Example 16: 9,9',9"-(benzene-1,3,5-triyltrimethylylidene)tris(2,7-dibromo-9H-fluorene) (8)

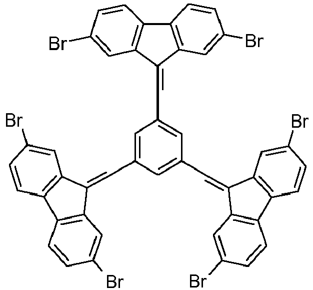

To a solution of benzene-1,3,5-tricarboxaldehyde (0.49 g, 3.0 mmol), 2,7-dibromofluorene (3.65 g, 11.27 mmol) and tetrabutylammonium bromide (0.52 g, 1.61 mmol) in toluene (21 mL), aqueous 40% NaOH solution (21 mL) was added and the obtained mixture was stirred at room temperature for 2 hours. Green crystals, formed upon standing, were filtered off and washed with water and later twice with ethanol to give the corresponding compound 8 (2.23 g, 68%), which was used in the next reaction without further purification.
Anal. calcd for C₄₈H₂₄Br₆: C, 53.38; H, 2.24; found: C, 53.58; H, 2.43.

### Example 17: 9,9',9"-(benzene-1,3,5-triyltrimethylylidene)tris[N,N,N',N'-tetrakis(4-methoxyphenyl)-9H-fluorene-2,7-diamine] (HTM-9)

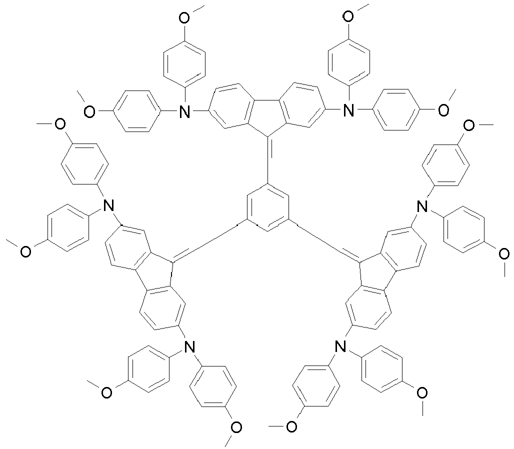

A solution of 8 (1.08 g, 1.0 mmol), 4,4'dimethoxydiphenylamine (2.06 g, 9.0 mmol) and toluene (17 mL) was purged with argon for 30 minutes. Afterwards, palladium(II) acetate (0.007 g, 0.03 mmol), tri-tert-butylphosphonium tetrafluoroborate (0.012 g, 0.04 mmol) and sodium tert-butoxide (0.86 g, 9.0 mmol) were added and the solution was refluxed under argon atmosphere for 5 hours. After cooling to room temperature, reaction mixture was filtered through Celite, 35 mL of distilled water were added and extraction was done with ethyl acetate. The organic layer was dried over anhydrous Na₂SO₄, filtered, and solvent evaporated. The crude product was purified by column chromatography using 7:18 v/v tetrahydrofuran/n-hexane as an eluent to collect HTM-9 as a light brown red solid (0.59 g, 26.4 %).
¹H-NMR (400 MHz, CDCl₃): δ = 7.40-6.87 (m, 32H), 6.79 (d, J=8.7 Hz, 16H), 6.66 (d, J=7.7 Hz, 12H), 6.47 (d, J=8.7 Hz, 12H), 3.68 (s, 18H), 3.55 (s, 18H);
¹³C-NMR (100 MHz, CDCl₃): δ = 155.48, 140.63, 137.63, 136.50, 136.20, 128.51, 126.37, 126.10, 126.05, 116.69, 114.65, 114.37, 113.79, 55.40, 55.22.
Anal. calcd for C₁₃₂H₁₀₈N₆O₁₂: C, 80.47; H, 5.52, N, 4.27; found: C, 80.58; H, 5.66; N, 4.10.

### Example 18: 3,7-bis[(2,7-dibromo-9H-fluoren-9-ylidene)methyl]-10-methyl-10H-phenothiazine (9)

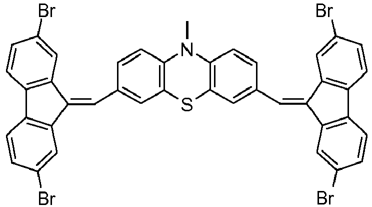

To 10-methylphenothiazin-3,7-dicarbaldehyde (0.81 g, 3.0 mmol), 2,7-dibromofluorene (2.43 g, 7.5 mmol) and tetrabutylammonium bromide (0.35 g, 1.1 mmol) dissolved in toluene (16 mL), aqueous 40% NaOH solution (16 mL) was added and the obtained mixture was stirred at room temperature for 3 hours. Green crystals of compound 9 (1.6 g, 73%), formed upon standing, were filtered off and washed with water and later once with ethanol and once with acetone; m.p. 197-200 °C.
¹H-NMR (400 MHz, CDCl₃): δ = 7.98 (d, J=1.6 Hz, 1 H), 7.86 (d, J=1.6 Hz, 1 H), 7.78 (d, J=1.6 Hz, 1 H), 7.66-7.15 (m, 17H), 3.54 (s, 3H);
¹³C-NMR (100 MHz, CDCl₃): δ = 147.88, 146.68, 142.22, 141.17, 138.84, 138.05, 137.43, 136.71, 135.22, 133.69, 131.16, 130.85, 130.63, 129.89, 129.84, 129.68, 127.81, 127.29, 125.58, 123.47, 123.28, 121.81, 121.12, 120.98, 120.90, 120.70, 35.31.
Anal. calcd for C₄₁H₂₃Br₄NS: C, 55.88; H, 2.63, N, 1.59; found: C, 55.71; H, 2.76; N, 1.43.

### Example 19: 9,9'-[(10-methyl-10H-phenothiazine-3,7-diyl)dimethylylidene]bis[N,N,N',N'-tetrakis(4-methoxyphenyl)-9H-fluorene-2,7-diamine] (HTM-10)

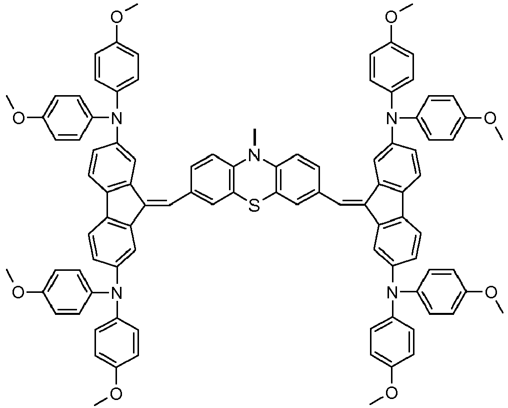

A solution of 9 (1.47 g, 1.0 mmol), 4,4'dimethoxydiphenylamine (1.38 g, 6.0 mmol) and toluene (14 mL) was purged with argon for 30 minutes. Afterwards, palladium(II) acetate (0.004 g, 0.02 mmol), tri-tert-butylphosphonium tetrafluoroborate (0.008 g, 0.027 mmol) and sodium tert-butoxide (0.58 g, 6.0 mmol) were added and the solution was refluxed under argon atmosphere for 5 hours. After cooling to room temperature, reaction mixture was filtered through Celite, 30 mL of distilled water were added and extraction was done with ethyl acetate. The organic layer was dried over anhydrous Na₂SO₄, filtered, and solvent evaporated. The crude product was purified by column chromatography using 1:4 v/v acetone/n-hexane as an eluent to collect HTM-10 as an orange solid (0.93 g, 38 %).
¹H-NMR (400 MHz, CDCl₃): δ = 7.51-7.45 (m, 2H), 7.38 (d, J=8.2Hz, 4H), 7.32-7.27 (m, 2H), 7.21-7.13 (m, 4H), 7.05 (d, J=8.4 Hz, 8H), 7. 0-6.72 (m, 30H), 6.24 (d, J=8.6 Hz, 2H), 3.79 (s, 12H), 3.76 (s, 12H), 3.14 (s, 3H);
¹³C-NMR (100 MHz, CDCl₃): δ = 155.30, 155.16, 147.23, 146.88, 144.55, 141.51, 140.76, 137.31, 134.99, 134.87, 132.81, 130.26, 128.42, 127.93, 126.04, 125.73, 125.64, 122.63, 122.51, 122.36, 119.30, 118.86, 114.58, 114.43, 113.24, 55.45, 55.39, 34.76.
Anal. calcd for C₉₇H₇₉N₅O₈S: C, 79.00; H, 5.40, N, 4.75; found: C, 79.24; H, 5.31; N, 4.87.

### Example 20: 2,5-bis[(2,7-dibromo-9H-fluoren-9-ylidene)methyl]thiophene (10)

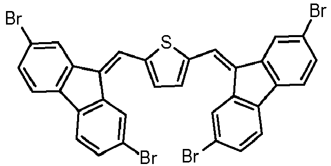

To 2,5-thiophenedicarboxaldehyde (0.42 g, 3.0 mmol), 2,7-dibromofluorene (2.43 g, 7.50 mmol) and tetrabutylammonium bromide (0.35 g, 1.09 mmol) dissolved in toluene (15 mL), aqueous 40% NaOH solution (15 mL) was added and the obtained mixture was stirred at room temperature for 1 hour. Light green crystals of compound 10 (1.3 g, 58%), formed upon standing, were filtered off and washed with water and ethanol; m.p. 216-218 °C.
¹H-NMR (400 MHz, CDCl₃, DMSO-d₆): δ = 7.90-7.12 (m, 16H);
¹³C-NMR (100 MHz, CDCl₃, DMSO-d₆): δ = 142.27, 137.58, 135.14, 127.52, 123.14, 122.27. Anal. calcd for C₃₂H₁₆Br₄S: C, 51.10; H, 2.14; found: C, 51.27; H, 2.30.

### Example 21: 9,9'-(thiene-2,5-diyldimethylylidene)bis[N,N,N',N'-tetrakis(4-methoxyphenyl)-9H-fluorene-2,7-diamine] (HTM-11)

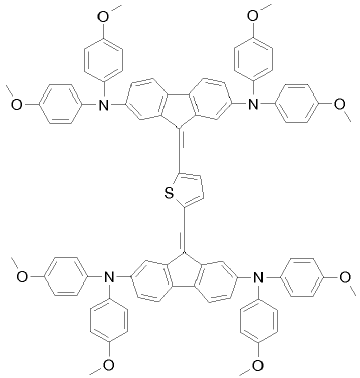

A solution of 10 (0.75 g, 1.0 mmol), 4,4'dimethoxydiphenylamine (1.38 g, 6.0 mmol) and toluene (12 mL) was purged with argon for 30 minutes. Afterwards, palladium(II) acetate (0.004 g, 0.02 mmol), tri-tert-butylphosphonium tetrafluoroborate (0.008 g, 0.027 mmol) and sodium tert-butoxide (0.58 g, 6.0 mmol) were added and the solution was refluxed under argon atmosphere for 5 hours. After cooling to room temperature, reaction mixture was filtered through Celite, 30 mL of distilled water were added and extraction was done with ethyl acetate. The organic layer was dried over anhydrous Na₂SO₄, filtered, and solvent evaporated. The crude product was purified by column chromatography using 3:22 v/v tetrahydrofuran/n-hexane as an eluent to collect HTM-11 as a dark brown solid (0.36 g, 27 %).
¹H-NMR (400 MHz, CDCl₃): δ = 7.98 (d, J=1.8 Hz, 1 H), 7.67 (d, J=2.0 Hz, 1 H), 7.37 (dd, J=8.4 Hz, J=1.8 Hz, 4H), 7.28-7.21 (m, 2H), 7.13-7.03 (m, 7H), 7.02-6.89 (m, 9H), 6.88-6.77 (m, 12H), 6.74-6.66 (m, 12H), 3.77 (s, 9H), 3.76 (s, 6H), 3.60 (s, 9H),
¹³C-NMR (100 MHz, CDCl₃): δ = 155.60, 155.31, 155.02, 147.53, 147.10, 146.84, 141.55, 141.38, 140.99, 140.83, 140.63, 139.17, 136.95, 135.82, 135.03, 133.97, 133.02, 130.69, 126.45, 125.67, 125.32, 121.34, 120.95, 119.21, 118.52, 114.61, 55.45, 55.40, 55.21.
Anal. calcd for C₈₈H₇₂N₄O₈S: C, 78.55; H, 5.39, N, 4.16; found: C, 78.39; H, 5.50; N, 4.00.

### Example 22: 4-[(2,7-dibromo-9H-fluoren-9-ylidene)methyl]-N,N-bis{4-[(2,7-dibromo-9H-fluoren-9-ylidene)methyl]phenyl}aniline (11)

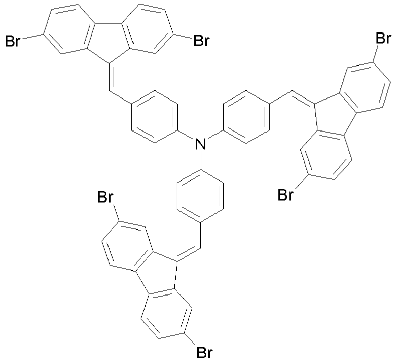

To tris(4-formylphenyl)amine (0.74 g, 2.24 mmol), 2,7-dibromofluorene (2.50 g, 7.85 mmol) and tetrabutylammonium bromide (0.39 g, 1.21 mmol) dissolved in toluene (16 mL), aqueous 40% K₂CO₃ solution (16 mL) was added and the obtained mixture was refluxed for 30 min. After cooling to room temperature, 50 mL of distilled water were added. Orange crystals of compound 11 (2.10 g, 76%), formed upon standing, were filtered off and washed with methanol, m.p. 304-305 °C.
¹H-NMR (400 MHz, THF-d₈): δ =8.14 (d, J=1.6 Hz, 3H), 8.0 (s, 3H), 7.94 (d, J=1.6 Hz, 3H), 7.80-7.72 (m, 6H), 7.68 (d, J=8.6 Hz, 6H), 7.57-7.50 (m, 6H), 7.48 (d, J=8.6 Hz, 6H);
Anal. calcd for C₆₀H₃₃Br₆N: C, 57.78; H, 2.67, N, 1.12; found: C, 57.96; H, 2.79; N, 1.01.

### Example 23: 9,9',9"-[nitrilotris(benzene-4,1-diylmethylylidene)]tris[N,N,N',N'-tetrakis(4-methoxyphenyl)-9H-fluorene-2,7-diamine] (HTM-12)

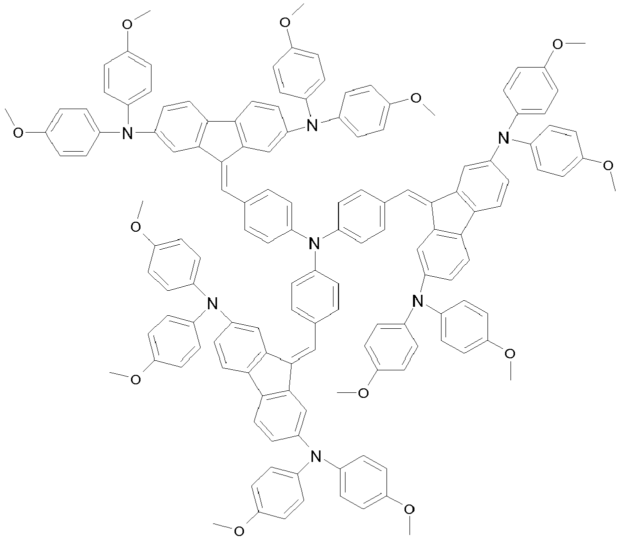

A solution of 11 (1.00 g, 0.80 mmol), 4,4'dimethoxydiphenylamine (1.65 g, 7.21 mmol) and toluene (15 mL) was purged with argon for 30 minutes. Afterwards, palladium(II) acetate (0.005 g, 0.02 mmol), tri-tert-butylphosphonium tetrafluoroborate (0.01 g, 0.032 mmol) and sodium tert-butoxide (0.69 g, 7.21 mmol) were added and the solution was refluxed under argon atmosphere for 6 hours. After cooling to room temperature, reaction mixture was filtered through Celite, 50 mL of distilled water were added and extraction was done with ethyl acetate. The organic layer was dried over anhydrous Na₂SO₄, filtered, and solvent evaporated. The crude product was purified by column chromatography using 0.5:7:17.5 v/v acetone/n-hexane/toluene as an eluent to collect HTM-12 as a dark orange solid (1.00 g, 59 %).
¹H-NMR (400 MHz, CDCl₃): δ = 7.61 (s, 3H), 7.42 (dd, J=8.2 Hz, J=2.8 Hz, 6H), 7.38 (s, 3H), 7.32-7.22 (m, 9H), 7.06 (d, J=8.9 Hz, 12H), 6.99-6.89 (m, 18H), 6.82 (d, J=8.9 Hz, 12H), 6.68 (d, J=9.0 Hz, 18H), 3.79 (s, 18H), 3.54 (s, 18H);
¹³C-NMR (100 MHz, CDCl₃): δ = 155.31, 155.09, 147.29, 146.92, 146.41, 141.52, 141.44, 140.97, 137.37, 135.17, 134.86, 132.76, 130.72, 130.50, 126.79, 125.72, 125.14, 123.72, 123.09, 122.72, 119.812, 119.38, 114.58, 114.49, 114.02, 55.46, 55.31.
Anal. calcd for C₁₄₄H₁₁₇N₇O₁₂: C, 80.91; H, 5.52, N, 4.59; found: C, 81.08; H, 5.47; N, 4.71.

### Example 24: N,N'-bis(4-methoxyphenyl)-N,N'-diphenylbiphenyl-4,4'-diamine (12)

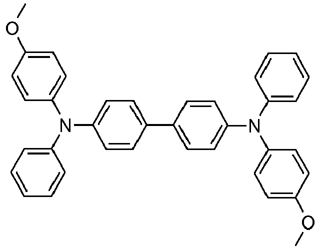

### 4,4'-{biphenyl-4,4'-diylbis[(4-methoxyphenyl)imino]}dibenzaldehyde (13)

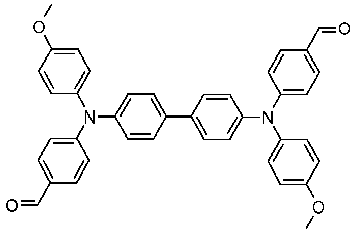

12 and 13 were synthesized according to the known procedure (Yen-Ju Cheng, Ming-Hung Liao, Hung-Min Shih, Ping-I Shih, Chain-Shu Hsu; Exciplex Electroluminescence Induced by Cross-Linked Hole-Transporting Materials for White Light Polymer Light-Emitting Diodes; Macromolecules, 2011, 44 (15), pp 5968-5976).

### Example 25: N,N'-bis{4-[(2,7-dibromo-9H-fluoren-9-ylidene)methyl]phenyl}-N,N'-bis(4-methoxyphenyl)biphenyl-4,4'-diamine (14)

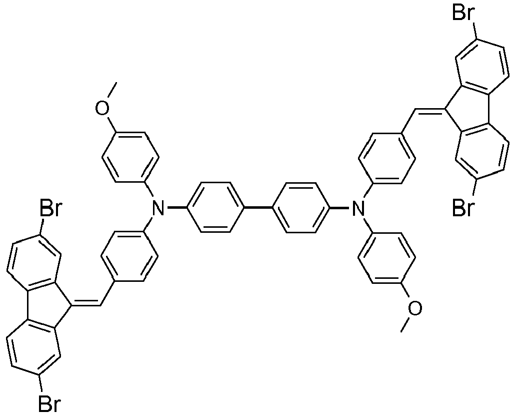

To dialdehyde 13 (1.5 g, 2.48 mmol), 2,7-dibromofluorene (2.0 g, 6.20 mmol) and tetrabutylammonium bromide (0.28 g, 0.89 mmol) dissolved in toluene (20 mL), aqueous 40% NaOH solution (18 mL) was added and the obtained mixture was refluxed for 45 min. After cooling to room temperature, 50 mL of distilled water were added and extraction was done with ethyl acetate. The combined organic layer was washed with distilled water, dried over anhydrous Na₂SO₄ and filtered. After evaporation of the solvent, the crude product was purified by column chromatography using 1:4 v/v acetone/n-hexane as an eluent to collect compound 14 as an orange crystals (2.57 g, 85%).
¹H-NMR (400 MHz, CDCl₃): δ = 7.93 (d, J=1.6 Hz, 2H), 7.79 (d, J=1.4 Hz, 2H), 7.56-7.34 (m, 20H), 7.24-7.15 (m, 6H), 7.12 (d, J=8.6 Hz, 4H), 6.90 (d, J=9.0Hz, 4H), 3.80 (s, 6H);
¹³C-NMR (100 MHz, CDCl₃): δ = 156.69, 148.64, 146.12, 141.29, 139.70, 138.62, 138.00, 136.39, 134.99, 132.64, 130.86, 130.60, 130.48, 130.23, 127.90, 127.78, 127.41, 127.01, 124.10, 123.23, 120.97, 120.85, 120.74, 120.54, 114.93, 55.44.
Anal. calcd for C₆₆H₄₄Br₄N₂O₂: C, 65.15; H, 3.65, N, 2.30; found: C, 65.39; H, 3.73; N, 2.53.

### Example 26: 9,9'-(biphenyl-4,4'-diylbis{[(4-methoxyphenyl)imino]benzene-4,1-diylmethylylidene})bis[N,N,N',N'-tetrakis(4-methoxyphenyl)-9H-fluorene-2,7-diamine] (HTM-13)

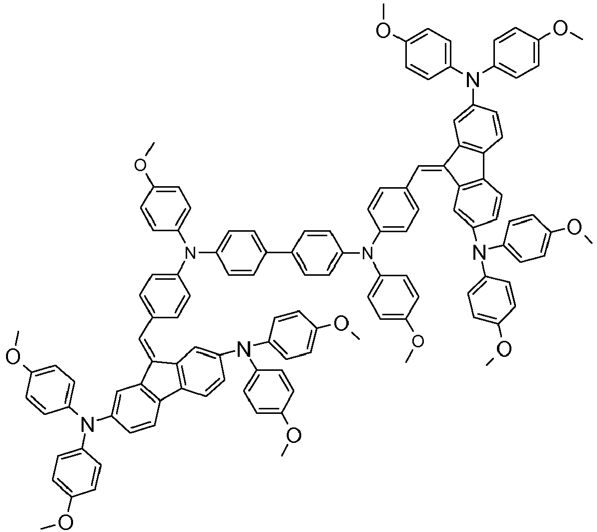

A solution of 14 (1.0 g, 0.82 mmol), 4,4'dimethoxydiphenylamine (1.13 g, 4.91 mmol) and toluene (12 mL) was purged with argon for 30 minutes. Afterwards, palladium(II) acetate (0.004 g, 0.02 mmol), tri-tert-butylphosphonium tetrafluoroborate (0.004 g, 0.02 mmol) and sodium tert-butoxide (0.47 g, 4.93 mmol) were added and the solution was refluxed under argon atmosphere for 5 hours. After cooling to room temperature, reaction mixture was filtered through Celite, 30 mL of distilled water were added and extraction was done with ethyl acetate. The organic layer was dried over anhydrous Na₂SO₄, filtered, and solvent evaporated. The crude product was purified by column chromatography using 0.5:10:14.5 v/v acetone/n-hexane/toluene as an eluent to collect HTM-13 as a dark orange solid (0.66 g, 44 %). ¹H-NMR (400 MHz, CDCl₃): δ = 7.58 (d, J=2.0 Hz, 2H), 7.47-7.34 (m, 8H), 7.33 (d, J=2.0 Hz, 2H), 7.26 (s, 2H), 7.21 (d, J=8.6 Hz, 4H), 7.11-6.90 (m, 28H), 6.86 (d, J=9.0 Hz, 4H), 6.82 (d, J=9.0 Hz, 8H), 6.77 (d, J=8.6 Hz, 4H), 6.72 (d, J=9.0 Hz, 8 H), 3.81 (s, 6H), 3.79 (s, 12H), 3.61 (s, 12H);
¹³C-NMR (100 MHz, CDCl₃): δ = 156.29, 155.26, 155.12, 147.44, 147.18, 146.89, 146.53, 141.45, 140.95, 140.18, 137.48, 134.86, 134.56, 134.10, 132.76, 130.24, 129.52, 127.31, 127.18, 127.02, 125.69, 125.35, 123.00, 122.52, 119.31, 114.75, 114.56, 114.46, 113.99, 55.49, 55.31.
Anal. calcd for C₁₂₂H₁₀₀N₆O₁₀: C, 80.95; H, 5.57, N, 4.64; found: C, 81.11; H, 5.69; N, 4.52.

### Example 27: Dye-sensitized Solar Cells

Dye sensitized solar cells were prepared according to the following procedure. A TiO₂ blocking layer was prepared on a fluorine-doped tin oxide (FTO)-covered glass substrate using spray pyrolysis (cf. B. Peng, G. Jungmann, C. Jager, D. Haarer, H. W. Schmidt, M. Thelakkat, Coord. Chem. Rev. 2004, 248, 1479). Next, a TiO₂ paste (Dyesol), diluted with terpineol, was applied by screen printing, resulting in a film thickness of approximately 1.7 µm. All films were then sintered for 45 min at 450 °C, followed by treatment in a 40 mM aqueous solution of TiCl₄ at 60 °C for 30 min, followed by another sintering step. The prepared samples with TiO₂ layers were pretreated with a 5 mM solution of the additive 2-(p-butoxyphenyl)acetohydroxamic acid sodium salt ("ADD1") in ethanol (this additive is described on page 52 of WO 2012/ 001 628 A1 as "Example No. 6"). The electrodes were then dyed in 0.5 mM dye solution in CH₂Cl₂. The respective hole-transport material according to the present invention was applied by spin-coating from a solution in DCM (200 mg/mL) also containing 20 mM Li(CF₃SO₂)₂N. Fabrication of the device was completed by evaporation of 200 nm of silver as the counter electrode. The active area of the solar cell was defined by the size of these contacts (0.13 cm²), and the cells were masked by an aperture of the same area for measurements.

The Current-voltage characteristics for all cells were measured with a Keithley 2400 under 1000 W/m², AM 1.5G conditions (LOT ORIEL 450 W). The results are given in table 1.

| Hole-transport material | Iₚ in eV | µ₀ in cm²V⁻¹S⁻¹ | µ₁ in cm²V⁻¹S⁻¹ | η in % |
|---|---|---|---|---|
| HTM-1 (Example 2) | 5.00 | - | - | 1.9 |
| HTM-2 (Example 3) | 4.98 | - | - | 2.3 |
| HTM-3 (Example 5) | 5.04 | 7 · 10⁻⁶ | 2.4 · 10⁻⁴ | 0.9 |

Table 1: Current-voltage characteristics of the dye-sensitzed solar cells with the hole-transport materials according to the present invention, Iₚ stands for solid state ionization potential of the hole-transport material, µ₀ for the charge-carrier mobility at 0 V/cm, µ₁ for the charge-carrier mobility at 6.4·10⁵ V/cm (both mobilities measured as neat film) and η for the solar-to-electric power conversion efficiency, a "-" indicates that the respective value was not measured.

The solid state ionization potential (Iₚ) of the hole-transport materials was measured by the electron photoemission in air method as described by Kirkus et al., Synthetic Metals 159 (2009) 729-734. The hole-transport materials were dissolved in CHCl₃ and coated on Al plates to form a layer of 0.5 to 1 µm thickness. The Al plates were pre-coated with a methylmethacrylate and methacrylic acid copolymer adhesive layer of a thickness of about 0.5 µm. The photoemission experiments were carried out in air. The samples were illuminated with monochromatic light from a deuterium lamp equipped with a quartz monochromator. The power of the incident light beam was 2 to 5 · 10⁻⁸ W. A negative voltage of -300 V was applied to the sample substrate. The counter-electrode with the 4.5×15 mm² slit for illumination was placed at 8 mm distance from the sample surface. The counter-electrode was connected to the input of the BK2-16 type electrometer, working in the open input regime, for the photocurrent measurement. A photocurrent of 10⁻¹⁵ to 10⁻¹² A was measured in the circuit under illumination. The square root of the photocurrent value was plotted against the energy of the photon. The linear part of this dependency was extrapolated to zero photocurrent. The corresponding photon energy was used as value for Iₚ.

To measure the charge carrier mobility (µ) the hole-transport materials were spin-coated from solution on polyester films with a conductive Al layer. The layer thickness was in the range of 5-10 µm. The hole mobility was measured by xerographic time of flight technique (XTOF) as described by Montrimas et al. Journal of Physics 6 (1966) 569. The electric field was created by positive corona charging. The charge carriers were generated at the layer surface by illumination with pulses of a nitrogen laser with a pulse duration of 2 ns and a wavelength of 337 nm. The layer surface potential decrease as a result of pulse illumination was 1 to 5 % of the potential before illumination. The capacitance probe that was connected to the wide frequency band electrometer measured the speed of the surface potential decrease (dU/dt). The transit time tₜ was determined by the kink on the curve of the dU/dt transient in double logarithmic scale. The drift mobility was calculated by the formula µ=d²/U₀tₜ, wherein d is the layer thickness and U₀ is the surface potential at the moment of illumination.

### Example 28: Perovskite Solar Cells

Perovskite Solar Cells were prepared according to the following procedure. A TiO₂ blocking layer was prepared in the same way as in Example 27. A TiO₂ scaffold was deposited by spin-coating a TiO₂ paste (Dyesol) diluted with ethanol. The two layers were sintered at 450 °C for 30 min. After cooling the substrates down to room temperature, a perovskite layer was prepared by spin-coating a 40 wt.-% solution of CH₃NH₃l:PbCl₂ (molecular ration 3:1) in DMF (N,N-Dimethylformamide) and annealing the resulting layer for 30 min in an oven at 110°C. The hole-transport material according to the present invention was deposited by spin-coating a solution in chlorobenzene containing also lithium bis(trifluoromethylsulfonyl)imide (LiTFSi) and tert-butylpyridine (tBP). The counter electrodes consisted of 50 nm gold layers evaporated through a mask, defining the active area of the solar cell by the size of these contacts (0.13 cm²). For measurements, the cells were masked by an aperture of the same area.

The hole-transport materials and the solar cells made thereof where characterized as described for example 27. The results are given in table 2.

| Hole-transport material | Ip in eV | µ₀ in cm²V⁻¹S⁻¹ | µ₁ in cm²V⁻¹S⁻¹ | η in % |
|---|---|---|---|---|
| HTM-1 (Example 2) | 5.00 | - | - | 2.7 |
| HTM-2 (Example 3) | 4.98 | - | - | 2.0 |
| HTM-3 (Example 5) | 5.04 | 7 · 10⁻⁶ | 2.4 · 10⁻⁴ | 0.5 |
| HTM-4 (Example 7) | 5.00 | 2.3 · 10⁻⁵ | 6 · 10⁻⁴ | 1.9 |
| HTM-5 (Example 9) | 5.00 | 1.4 · 10⁻⁵ | 6 · 10⁻⁴ | 2.0 |
| HTM-6 (Example 11) | 5.00 | 1.2 · 10⁻⁴ | 3 · 10⁻³ | 2.2 |
| HTM-7 (Example 13) | 5.02 | 2 · 10⁻⁵ | 7 · 10⁻⁴ | 6.6 |
| HTM-8 (Example 15) | 4.98 | 1.4 · 10⁻⁵ | 2 · 10⁻⁴ | 7.4 |
| HTM-9 (Example 17) | 5.02 | 7 · 10⁻⁶ | 4 · 10⁻⁴ | 6.1 |
| HTM-10 (Example 19) | 5.00 | 6 · 10⁻⁶ | 1.8 · 10⁻⁴ | 2.6 |
| HTM-11 (Example 21) | 5.00 | 8 · 10⁻⁵ | 1 · 10⁻³ | - |
| HTM-12 (Example 23) | 5.03 | - | - | - |
| HTM-13 (Example 26) | 4.96 | - | - | - |

Table 2: Current-voltage characteristics of the perovskite solar cells with the hole-transport materials according to the present invention, the measures have the same meaning as in table 1.

## Claims

1. A compound of general formula (I) 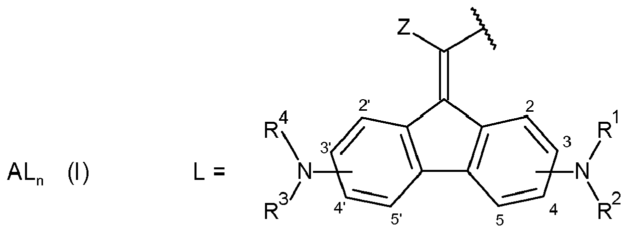 wherein
A is a group which connects all L moieties by a π-conjugated system which is formed by olefinic groups, aromatic groups, heteroaromatic groups, sandwich groups, atoms with unbound electron pairs, or combinations thereof,
Z is hydrogen, an alkyl group, an aryl group, or a heteroaryl group,
R¹, R², R³, R⁴ are independent of each other alkyl groups, aryl groups, or heteroaryl groups,
n is an integer of at least 2.

2. The compound according to claim 1, wherein the π-conjugated system in A is formed by one or more aromatic and/or heteroaromatic groups.

3. The compound according to claim 2, wherein the π-conjugated system in A is formed by benzene.

4. The compound according to any of the claims 1 to 3, wherein n equals 2.

5. The compound according to any of the claims 1 to 4, wherein R¹, R², R³, R⁴ are independent of each other alkoxyphenyl groups.

6. The compound according to any of the claims 1 to 5, wherein Z is hydrogen.

7. Use of the compound of general formula (I) according to any of the claims 1 to 6 in organic solar cells or organic optical sensors.

8. An organic solar cell or organic optical sensor comprising a compound of general formula (I) according to any of the claims 1 to 6.

9. The organic solar cell or organic optical sensor according to claim 8, wherein the organic solar cell or organic optical sensor further comprises a semiconducting metal oxide as electron transport material which is sensitized with a light-absorbing material.

10. The organic solar cell or organic optical sensor according to claim 9, wherein the metal oxide is TiO₂.

11. The organic solar cell or organic optical sensor according to claim 9 or 10, wherein the light-absorbing material is a Ru(II) dye, a perylene dye, a naphtalenemonoimid dye, or a quinolinium dye.

12. The organic solar cell or organic optical sensor according to claim 9 or 10, wherein the light-absorbing material is a perovskite absorber.
